# EUROPÄISCHE PATENTSCHRIFT

(11) **EP 1 108 402 B1**
(45) Veröffentlichungstag und Bekanntmachung des Hinweises auf die Patenterteilung: **20.09.2006**
(21) Anmeldenummer: 00203860.2
(22) Anmeldetag: 03.11.2000
(51) Int. Cl.: A61F 2/16

(54) **Fokussierfähige Intraokularlinse**
Accomodating intraocular lens
Lentille intraoculaire capable de s'accommoder

(30) Priorität: 14.12.1999 DE 19960136
(43) Veröffentlichungstag der Anmeldung: 20.06.2001
(73) Patentinhaber: Boehm, Hans-Georg, Dr. rer. nat., D-61476 Kronberg/Ts (DE)
(72) Erfinder: Boehm, Hans-Georg, Dr. rer. nat., D-61476 Kronberg/Ts (DE)

(56) Entgegenhaltungen:
- WO-A-96/15734
- WO-A-99/03427
- US-A- 5 609 630

## Beschreibung

### Stand der Technik:

Zur Behebung des grauen Stars (Linsentrübung) wird bei Kataraktoperationen die natürliche Augenlinse durch eine Kunstlinse ersetzt. Solche Kunstlinsen besitzen in der Regel die Form eines Paragraphenzeichens, wobei die abstehenden s-förmigen Bügel zu Ihrer Zentrierung im Hohlraum der entfernten Naturlinse dienen. Diese Kunstlinsen sind zwar in feuchtem Zustand sehr flexibel und können zum Einsetzen ins Auge durch einen möglichst kleinen Schlitz sogar gefaltet werden, doch sie sind nicht mehr konvex verformbar, wie dies zur Akkomodation bei natürlichen Augenlinsen der Fall ist. Eine Kunstlinse hat eben nur einen unveränderlichen Brennpunkt, deshalb muß eine Schärfe von vor oder danach liegenden Bildpunkten durch angepaßte Brillenlinsen angenähert werden. Die daraus resultierende unvermeidliche Unschärfe in den Übergangsbereichen bedingt einen erheblichen Verlust an Sehkomfort, den es durch eine verbesserte fokussierfähige Kunstlinse zu mindern gilt und die die Chance bietet, auf Korrekturbrillen ganz verzichten zu können.

Auch ohne diagnostizierte Linsentrübung können später einmal gut fokussierenden Kunstlinsen, in gesunde Augen eingesetzt, die kostspieligen Laserabtragungen der Hornhaut zur Beseitigung starker Kurzsichtigkeit ersetzen und einer Altersweitsicht vorbeugen.

Statt durch Änderung der Konvexität steht als Alternative zur Akkomodation mit Kunstlinsen nur eine Änderung des Linsenabstandes von der Netzhaut (Versatz) durch den kontrahierenden Ringmuskel zur Verfügung, vorausgesetzt dieser blieb nach Entfernung der Naturlinse unbeschädigt und aktiv.

Fokussierfähige Kunstlinsen für Augen mit zwei Knickgelenken an den Zentrierfedern sind bereits aus dem Dokument WO 99 03427 A (ISRAEL HENRY M) 28. Januar 1999 (1999-01-28) bekannt. Weil diese Knickgelenke nahezu parallel zur Linsenebene ausgerichtet sind, erfordert dies eine ungleich größere Kontraktionskraft des Ringmuskels, um eine Axialverschiebung der Linse zu erzielen, als wie dies mit einem ideal um 50 Grad gegen die optische Linsenachse schräg gestelltem Mittelteil zwischen den beiden Knickgelenken der Zentrierfedern der Fall ist.

Weitere Kunstlinsen sind aus WO 96 15734 A (ACUITY ISRAEL LIMITED; ISTAEL HENRY M (IL) 30. Mai 1996 (1996-05-30) und US-A-5 609 630 (CROZAFON PHILIPPE) 11. März 1997 (1997-03-11) bekannt.

### Problemlösung und Beschreibung:

Diese Aufgabe wird durch z-ähnliche Zentrierfedern gemäß Anspruch 1 gelöst.

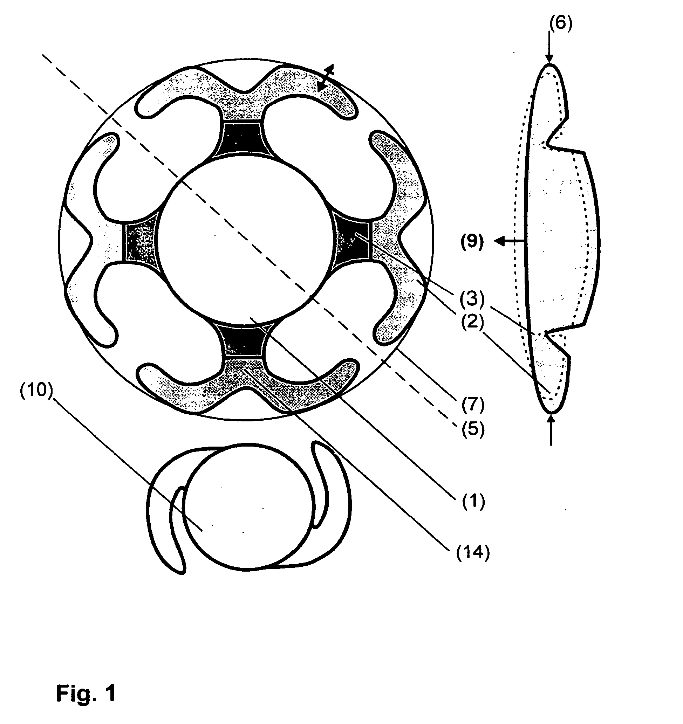

Fig. 1: zeigt eine fokussierfähige Kunstlinse mit nur einem Knickgelenk, wie sie der CUMMING'schen Linse (allgemeiner Stand der Technik) nachempfunden ist. Bei dieser Kunstlinse blieb die Empfindlichkeit bei Stauchung und der Versatz (9) noch unbefriedigend.

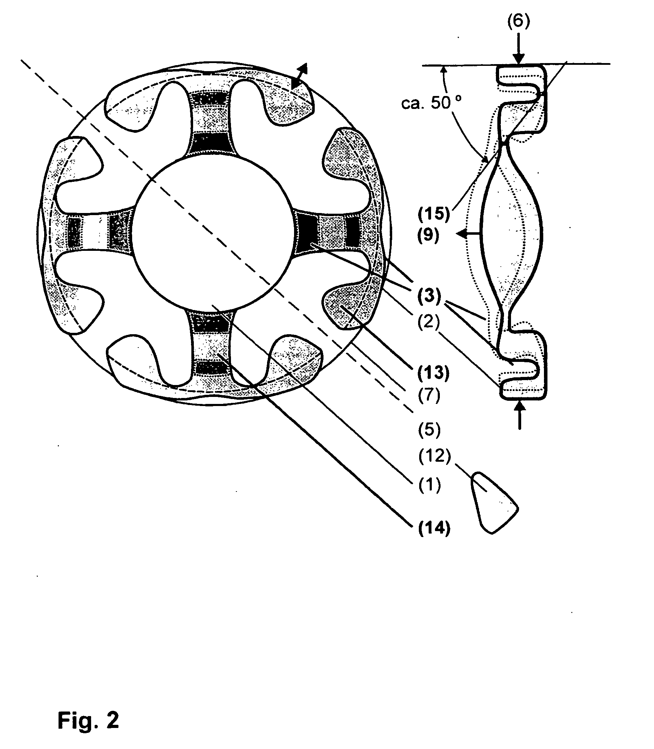

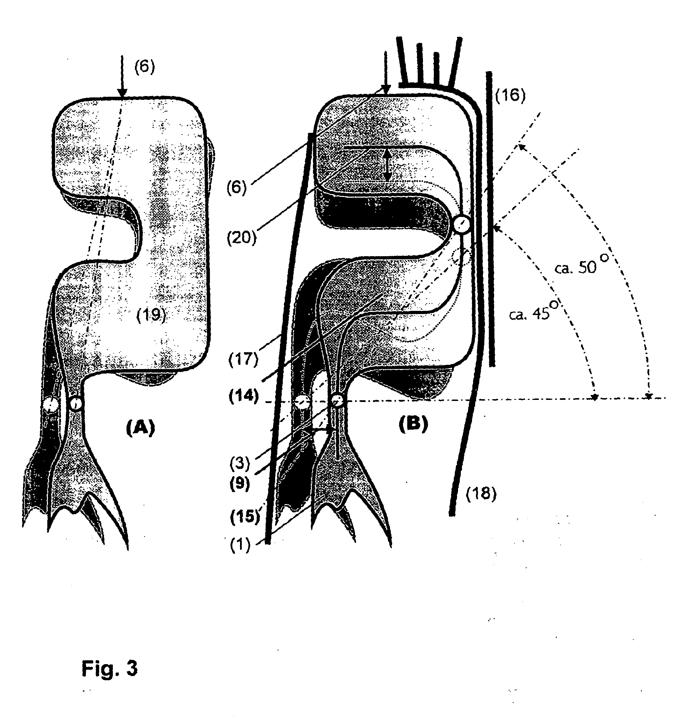

Fig. 2: Durch z-ähnliche Zentrierfedern (2) mit zwei Knickgelenken (3) am Steg (14) wird erfindungsgemäß jedoch der Versatz (9) wirkungsvoll vergrößert. Der Mittelteil des Steges der Zentrierfedern ist quaderförmig ausgebildet, damit seine Vorderseite an der vorderen Kapselsackmembran (17 in Fig. 3) und seine Rückseite an der vorderen Glaskörpergrenzmembran (16 in Fig. 3) anliegt. Dies unterstützt die Rückführung der Kunstlinse durch den bei einer Dilatation meridional gedehnten Kapselsack.

Die Stützlappen (13) am Ende der Zentrierfedern schmiegen sich mit ihrer Rückseite an die vordere Glaskörpergrenzmembran an und verhindern das alleinige Einknicken nur des äußeren Knickgelenks.

Bei entspanntem Ringmuskel strecken sich die gestauchten, elastischen Zentrierfedern nach einer Dilatation selbsttätig in ihre ursprüngliche Ausgangsform zurück und ziehen dabei die Kunstlinse wieder in ihre Ruheposition. Beim üblicherweise verwendeten weichelastischen Linsenmaterial sind die Rückstelleigenschaften der Zentrierfedern zur schnelleren Rückbildung einer Deformation durch eine partielle chemische oder physikalisch Behandlung (bspw durch UV-Bestrahlung stärker vernetzt) oder durch eine partielle feuchtigkeitsabweisende Oberflächenversiegelung (reduziert den weichmachenden Flüßigkeitseinfluß) zusätzlich versteift.

Fig. 3: zeigt die Verformung einer z-ähnlichen Zentrierfeder bei Kontraktion des Ringmuskels. Der Winkel der gedachten schrägen Verbindungslinie (15) zwischen den beiden Knickgelenken und der optischen Achse der Kunstlinse beträgt je nach Deformation zwischen ca. 45 bis 50 Grad, damit ist in diesem Bereich die maximale, dort auch nahezu lineare Empfindlichkeit dieses Akkomodationsapparates genutzt.

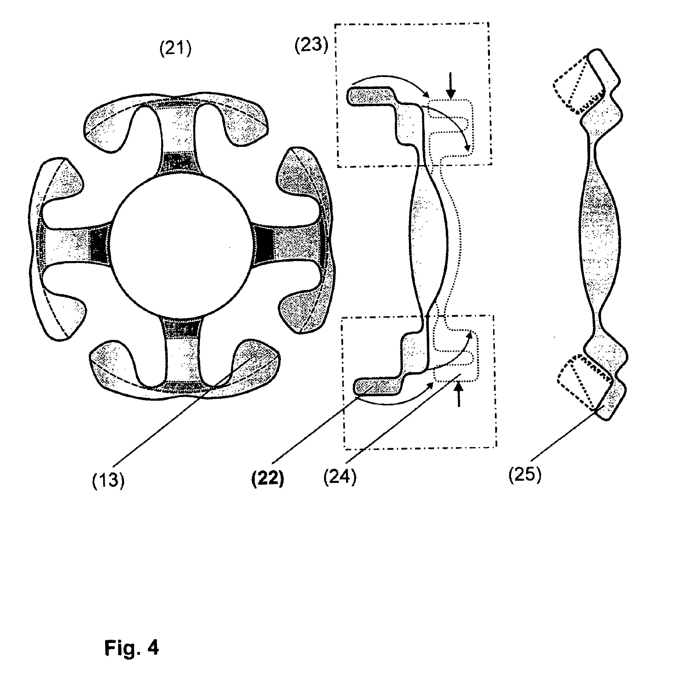

Fig. 4: zeigt die Herstellung einer Kunstlinse mit gestreckten Zentrierfedern in hartem Aggregatzustand, also noch bevor sie im Wasserdampf weichelastisch wird. Bei einer Implantation, in z-ähnliche Form gestaucht, stellt sich die Kunstlinse durch diese stärkere Verformung somit schneller nach einer Dilatation in ihre Ruheposition zurück.

### Bezeichnungen :

- Fig. 1: Fokussierfähige Kunstlinse mit je einem Knickgelenk (3) am Steg (14) ihrer Zentrierfedern, dem CUMMING-Prinzip nachempfunden
(1) Kunstlinse
(2) vier vom Rand (1) der Kunstlinse abstehende Zentrierfedern
(3) Knickgelenk am Ansatz der Zentrierfedern
(5) Faltlinie zum Zusammenklappen der Kunstlinse vor dem Einschieben in den Kapselsack (7)
(6) Angriffsumfang des kontrahierbaren Ringmuskels
(7) Kapselsack, verbleibender Hohlraum nach Entfernung der getrübten Naturlinse
(9) Versatz der Kunstlinse bei Kontraktion des Ringmuskels (6), Änderung des Linsenabstandes von der Netzhaut
(10) bisher benutzte Form nicht fokussierender Kunstlinsen mit zwei s-förmigen Zentrierfedern, verkleinert dargestellt
(14) Steg einer Zentrierfeder
- Fig. 2: Fokussierfähige Kunstlinse mit je zwei Knickgelenken (3) am Steg (14) der z-ähnlichen Zentrierfedern
(1) Kunstlinse
(2) vier am Rand der Kunstlinse abstehende Zentrierfedern schmiegen sich federnd an den Kapselsackrand an und zentrieren die Kunstlinse
(3) je zwei Knickgelenke am schmalen Steg einer Zentrierfeder
(5) Faltlinie zum Zusammenklappen der Kunstlinse vor dem Einschieben
(6) Angriffsumfang des kontrahierbaren Ringmuskels
(7) Kapselsack, verbleibender Hohlraum nach Entfernung der getrübten Naturlinse
(9) Versatz der Kunstlinse bei Kontraktion des Ringmuskels (6), Änderung des Linsenabstandes von der Netzhaut
(12) Querschnitt eines Stützlappens (13) an den Enden einer Zentrierfeder
(13) Stützlappen gegen die vordere Glaskörpermembran angelehnt, verhindern das alleinige Einknicken des oberen Knickgelenkes
(14) nicht verformbarer Mittelteil des Steges einer Zentrierfeder
(15) gedachte Verbindungslinie zwischen den undeformierten Knickgelenken, ca. 40 Grad gegen die optische Achse der Kunstlinse geneigt
- Fig. 3: Verformung einer z-ähnlichen Zentrierfeder mit einem (A) oder zwei (B) Knickgelenken (3) bei Kontraktion des Ringmuskels (6)
(1) Kunstlinse
(3) Knickgelenke, als kleine Kreise dargestellt
(6) Angriffsumfang des Ringmuskels
(9) Versatz der Kunstlinse bei Kontraktion des Ringmuskels (6), Änderung des Linsenabstandes von der Netzhaut
(14) nicht verformbarer Mittelteil des Steges einer Zentrierfeder
(15) gedachte Verbindungslinie zwischen den undeformierten Knickgelenken
(16) vordere Glaskörpergrenzmembran, an der Rückseite der Zentrierfeder und ihres Stützlappens anliegend
(17) vordere Kapselsackmembran
(18) hintere Kapselsackmembran mit angedeuteten Zonulafasern
(19) z-ähnliche Zentrierfeder mit nur einem Knickgelenk, dadurch geringe Empfindlichkeit (Änderung des Versatzes gegen aufgewandte Stauchkraft)
(20) die gedachte Mittellinie einer ungestauchten Zentrierfeder ist durchgehend, die der gestauchten ist dagegen gepunktet gezeichnet
- Fig. 4: Die Kunstlinse ist in hartem Aggregatzustand mit gestreckten Zentrierfedern hergestellt.
(13) Stützlappen
(21) Aufsicht einer Kunstlinse in hartem Aggregatzustand
(22) Querschnitt von (21) mit gestreckten Zentrierfedern, die Stützlappen und die Enden der Zentrierfedern sind hier nicht eingezeichnet
(23) im strich-punktiertem Bereich: Zentrierfedern zur Verbesserung ihrer Rückstelleigenschaften durch partielle chemische oder physikalische Nachbehandlung zusätzlich versteift
(24) Zentrierfedern sind nach einer Implantation z-ähnlich gestaucht
(25) Stellung der Zentrierfedern vor dem Falten und Einschieben der Kuntlinse in den Kapselsack, die Stützlappen und die Enden der Zentrierfedern sind hier eingezeichnet

## Patentansprüche

1. Fokussierfähige Kunstlinsen als Ersatz für getrübte körpereigene Augenlinsen mit vom Linsenrand (1) abstehenden Zentrierfedern (2), wobei die Zentrierfedern (2) einen z-ähnlichen Querschnitt und am Steg jeder Zentrierfeder zwei Knickgelenke (3) aufweisen, von denen das eine nahe des Linsenrandes und das andere am äußeren Rand der Zentrierfeder angeordnet ist, wobei sich an dem am äußeren Rand der Zentrierfeder angeordnetem Knickgelenk jeweils ein im wesentlichen ringsegmentförmiger Zentrierfederabschnitt anschließt, an dessen freien Enden Stützlappen (13) radial nach innen abstehen und so das alleinige Einknicken nur des äußeren Knickgelenks bei der Kontraktion des Ringmuskels verhindern.

## Claims

1. Accommodating lO-lenses for replacement of muddy natural lenses and a border (1) having centralizing spring haptics (2) standing off therefrom having a z-similar cross section, having two pivots (3) one near to the lens border and the other on the outer ends of the spring haptics, having support lobes (13) on each side at the free outer ring shaped spring ends which are radially directed to the lens center to avoid only bending of the outer pivot induced by a contraction of the ciliary muscle.

## Revendications

1. lO lentilles accommodatives comme remplacement de lentilles naturelles troubles avec un bord (1) et des bras centralisants et élastiques (2) étant écartés de celui-là, avec une forme z-semblable de coupe transversale, avec deux joints (3) dont l'un se trouve tout près du bord de la lentille et l'autre au bout extérieure du bras élastique, avec des lobes de support (13) à chaque côté des deux bouts extérieures libres des bras élastiques montrant vers le centre de la lentille pour éviter le joint extérieure d'être plié seule résultant par une contraction du muscle ciliaire.
